(19) Europäisches Patentamt
European Patent Office
Office européen des brevets

(11) **EP 3 764 898 B1**

(12) **EUROPEAN PATENT SPECIFICATION**

(45) Date of publication and mention
of the grant of the patent:
**19.03.2025 Bulletin 2025/12**

(21) Application number: **19718679.4**

(22) Date of filing: **17.04.2019**

(51) International Patent Classification (IPC):
$A61B\ 5/091\ ^{(2006.01)}$ $A61B\ 5/085\ ^{(2006.01)}$
$A61B\ 5/087\ ^{(2006.01)}$ $A61B\ 5/00\ ^{(2006.01)}$

(52) Cooperative Patent Classification (CPC):
**A61B 5/091; A61B 5/085; A61B 5/087;
A61B 5/0871; A61B 5/7239;** A61B 5/7264

(86) International application number:
**PCT/EP2019/059903**

(87) International publication number:
**WO 2019/201984 (24.10.2019 Gazette 2019/43)**

(54) **METHOD AND APPARATUS FOR DETERMINING AIRFLOW LIMITATION**

VERFAHREN UND VORRICHTUNG ZUR BESTIMMUNG DER LUFTSTROMBEGRENZUNG

PROCÉDÉ ET APPAREIL DE DÉTERMINATION D'UNE LIMITE DE FLUX D'AIR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2018 GB 201806243
24.04.2018 GB 201806647**

(43) Date of publication of application:
**20.01.2021 Bulletin 2021/03**

(73) Proprietor: **ArtiQ NV
3000 Leuven (BE)**

(72) Inventors:
• **DAS, Nilakash
4102 Binningen (BE)**
• **JANSSENS, Wim
3010 Leuven (BE)**
• **TOPALOVIC, Marko
3000 Leuven (BE)**

(74) Representative: **Winger
Mouterij 16 bus 101
3190 Boortmeerbeek (BE)**

(56) References cited:
US-A- 5 984 872 US-A1- 2010 147 305
US-A1- 2016 256 660

• SHUNSHOKU KANAE ET AL: "PARAMETER ESTIMATION OF NONLINEAR DIFFERENTIAL EQUATION MODELS OF RESPIRATORY SYSTEM BY USING NUMERICAL INTEGRATION TECHNIQUE", 5TH IFAC SYMPOSIUM ON MODELLING AND CONTROL IN BIOMEDICAL SYSTEMS 2003, MELBOURNE, AUSTRALIA, 21-23 AUGUST 2003, vol. 39, no. 1, 1 January 2006 (2006-01-01), pages 1288 - 1293, XP055587780, ISSN: 1474-6670, DOI: 10.3182/ 20060329-3-AU-2901.00208
• OSTADABBAS SARAH ET AL: "A passive quantitative measurement of airway resistance using depth data", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 5743 - 5747, XP032675019, DOI: 10.1109/ EMBC.2014.6944932

**Description**

**Field of the invention**

Embodiments of the present invention relate to methods and apparatus for determining airflow limitation, in particular for determining a parameter representative of airway resistance from spirometry data only.

**Background of the invention**

Spirometry is a method of detecting airflow limitation associated with obstructive lung diseases. In spirometry, two parameters, forced expiratory volume in 1 sec (FEV1) and the ratio FEV1/Forced vital capacity (FVC), are measured to indicate airflow limitation indirectly. Resistance to airflow is the physical basis behind airflow limitation and currently, there are three ways to estimate this resistance.

Airway resistance (Raw) which is measured during whole body-plethysmography, is determined as the ratio of alveolar driving pressure minus mouth pressure to flow rate. Plethysmography is performed using a plethysmograph, a bulky and non-portable apparatus which is generally only available in hospitals and requires specially trained operators. Another method aims to estimate respiratory impedance ($Z_{rs}$) which is measured during Forced Oscillation Technique (FOT). Respiratory impedance is determined as the opposition to sound waves in the respiratory system. A third method is the interrupter technique in which pressure changes during small interruptions of the expiratory flow are captured to calculate respiratory resistance (Rint).

All of the above-mentioned techniques aim to measure airway resistance during tidal breathing to derive resistance parameters that reflect airway patency independently from the forced maximal expiratory manoeuvre. Nevertheless, factors affecting airway resistance during these forced manoeuvres including lung elasticity and airway collapse, cannot be underestimated and often compose the main reason for airflow limitation and clinical symptoms. Measures of airway resistance obtained during the maximal expiration will provide better insight in these mechanisms, which are potentially amendable for intervention.

Moreover, access to the current technology for measuring airways resistance may not be available in primary care, especially body-plethysmography and FOT as they involve the use of expensive and bulky equipment.

In Flow Decay: A Novel Spirometric Index to Quantify Dynamic Airway Resistance, Oh et al., Respir. Care, Vol. 62 Issue 7 p928-935, the authors describe a model to measure the exponential decay in air flow during forced exhalation during spirometry to quantify the extent of dynamic airway obstruction. Flow decay is calculated as the slope of volume versus ln(1/flow) in mid-exhalation. However, the flow decay parameter does not explain the mechanics of forced expiration; it captures only the concavity of the flow-volume loop; and the flow decay parameter does not estimate airway resistance. US2010/147305,A1 describes a method using a forced oscillation technique for detecting expiratory flow limitation.

Therefore, there is a need for methods and apparatus for determining airway resistance which do not require use of a plethysmograph or other bulky, non-portable apparatus.

**Summary of the invention**

The invention is defined by the appended claims.

It is an object of the present invention to provide good methods and systems for determining airway resistance or airway limitation. It is an advantage of embodiments of the present invention to provide methods and systems for determining airway resistance or airway limitations that do not require complex or bulky measurement systems.

It is an advantage of embodiments of the present invention that the mouth pressure does not need to be measured since it is not used for deriving information regarding the airway resistance or airway limitation.

It is an advantage of embodiments of the present invention that the method can be based on information obtained during spirometry e.g. using only a spirometer. It is an advantage of embodiments of the present invention that no further bulky or complex apparatus needs to be used.

It is an advantage of embodiments of the present invention that a representative value for airway resistance or airway limitation can be derived that can be easily interpreted.

It is an advantage of embodiments of the present invention that the airway resistance or airway limitation can be represented by the measurement with a single value, allowing for an easy interpretation.

It is an advantage of embodiments of the present invention that the method and system can be used for analyzing forced expiration.

The object and optionally one or more advantages as described are obtained by methods and apparatus according to the claimed invention.

According to a first aspect, there is provided a method for analyzing forced expiration. The method comprises receiving forced expiration flow-volume data collected by a spirometer; fitting a second order differential equation in volume to the forced expiration flow-volume data; and determining a parameter representative of airway resistance in dependence upon, e.g. taking into account, a coefficient of a first order term in the differential equation. Where reference is made to a second order differential equation in volume, reference is made to a second order equation as function of the volume as variable. The independent variable in the differential equation is the time. In other words the change of volume as function of time, as well as the second differential of the volume as function of time are expressed in this equation.

It is an advantage of embodiments of the present invention that airway resistance can be quantified using a spirometer, a portable handheld device which is not restricted to use in hospitals by specially trained operators.

The second order differential equation may have the form according to equation (1) below:

$$\ddot{x} + 2\zeta\omega_n\dot{x} + \omega_n^2 x = \delta(t) \qquad (1)$$

wherein, in equation (1), $x$ is volume, $\dot{x}$ is the flow rate, $\zeta$ is a parameter representative of airway resistance, $\omega_n$ is a parameter representative of tissue elasticity and expiratory muscle force.

The data may comprise a first set of flow-volume data corresponding to forced expiration before a point of peak expiratory flow and fitting the second order differential equation to the data may comprise fitting at least one data point in the first set.

The data may comprise a second set of flow-volume data corresponding to forced expiration after a point of peak expiratory flow and fitting the second order differential equation to the data may comprise fitting at least one data point in the second set.

The method may comprise outputting the parameter.

The method may comprise determining a forced vital capacity based on the received flow-volume data and using the forced vital capacity as a boundary condition of the second order differential equation.

The method may comprise determining a peak expiratory flow value based on the received flow-volume data and using the peak expiratory flow value as a boundary condition of the second order differential equation.

According to a second aspect, there is provided a non-transitory computer-readable medium containing instructions which, when executed by a computer, cause the computer to carry out the steps of a method according to the first aspect.

According to a third aspect, there is provided a computer program product comprising instructions which, when executed by a computer, cause the computer to carry out the steps of a method according to the first aspect.

According to a fourth aspect, there is provided apparatus comprising a spirometer and a processor configured to receive forced expiration flow-volume data from the spirometer, wherein the processor is configured to perform the steps of fitting a second order differential equation in flow to the data; and determining a parameter representative of airway resistance in dependence upon a coefficient of a first order term in the differential equation.

The processor may be configured so that said fitting a second order differential equation in volume comprises fitting a second order differential equation having the form according to equation (1)

$$\ddot{x} + 2\zeta\omega_n\dot{x} + \omega_n^2 x = \delta(t) \qquad (1)$$

wherein, in equation (1), $x$ is volume, $\dot{x}$ is the flow rate, $\zeta$ is a parameter representative of airway resistance, $\omega_n$ is a parameter representative of tissue elasticity and expiratory muscle force, ), $t$ is time, and $\delta(t)$ is an impulsive force which occurs in time interval t resulting in Peak Expiratory Flow (PEF).

The processor may be configured so that said fitting comprises fitting the second order differential equation to at least one data point of a first set of flow-volume data corresponding to forced expiration before a point of peak expiratory flow.

The processor may be configured so that said fitting comprises fitting the second order differential equation to at least one data point of a second set of flow-volume data corresponding to forced expiration after a point of peak expiratory flow.

The processor further may be configured for determining a forced vital capacity based on the flow-volume data and using the forced vital capacity as a boundary condition of the second order differential equation.

The processor further may be configured for determining a peak expiratory flow value based on the flow-volume data and using the peak expiratory flow value as a boundary condition of the second order differential equation.

The processor may be configured for fitting the second order differential equation solely based on the flow-volume data.

The processor may be configured to provide the parameter as output.

The spirometer may comprise the processor.

The processor may be a software module.

The software module may be cloud-based.

Particular and preferred aspects of the invention are set out in the accompanying independent and dependent claims.

**Brief description of the drawings**

Further features of the present invention will become apparent from the examples and figures, wherein:

Fig. 1 is a flow chart of a method according to embodiments of the present invention;
Fig. 2 is a schematic diagram of a deflating balloon model;
Figs. 3a-3e show forced expiration flow-volume data obtained through spirometry plotted together with second-order differential equations fitted to the data using a method according to embodiments of the present invention;
Fig. 4a is a box plot of obstruction coefficient obtained using a method according to embodiments of the present invention for subjects having conditions ranging from none (Healthy) and mild airway obstruction (GOLD I COPD) to severe airway obstruction (GOLD IV COPD);
Fig. 4b is a box plot of airway resistance for subjects having conditions ranging from none (Healthy) and mild airway obstruction (GOLD I COPD) to severe airway obstruction (GOLD IV COPD);
Fig. 5a shows FEV1 as function of the obstruction coefficient (zeta);
Fig. 5b shows FEV1 as a function of airway resistance (Raw);
Fig. 6a is a schematic representation of a first apparatus according to embodiments of the present invention;
Fig. 6b is a schematic representation of a second apparatus according to embodiments of the present invention;
Fig. 6c is a schematic representation of a third apparatus according to embodiments of the present invention.

**Detailed description of preferred embodiments**

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein. In the drawings, like reference numerals indicate like features; and, a reference numeral appearing in more than one figure refers to the same element.

Where in embodiments of the present invention reference is made to a second order differential equation in volume, reference is made to a second order differential equation as function of the volume as variable, the derivative of the volume towards time also referred to as flow rate, and the second derivative of the volume towards time. In other words a relationship between the volume, the change of volume as function of time, as well as the second differential of the volume as function of time is used in embodiments of the present invention.

Where in embodiments of the present invention reference is made to fitting of the second order differential equation in volume to the forced expiration flow-volume data, reference is made to determining the second order differential equation in volume - and more particularly deriving the coefficients of the second order differential equation - based on the forced expiration flow-volume data obtained using e.g. a spirometer.

According to aspects of the present invention an apparatus is disclosed for determining a parameter representative of airway resistance, the apparatus comprising

- a spirometer for obtaining forced expiration flow-volume data,
- a processor configured to receive forced expiration flow-volume data from the spirometer,

wherein the processor is configured to perform the steps of:

(i) fitting a second order differential equation expressing the change of volume as function of time and the second differential of the volume as function of time to said flow-volume data and
(ii) determining a parameter representative of airway resistance in dependence upon a coefficient of a first order term in the obtained differential equation, said determining the parameter being based only on the spirometer data,

wherein the processor is further configured to output the parameter representative of the airway resistance.

Similarly, also a corresponding method is disclosed. Standard and optional features of methods and apparatus will further be illustrated with reference to the drawings, embodiments of the present invention not being limited thereto or thereby, but solely by the appended claims.

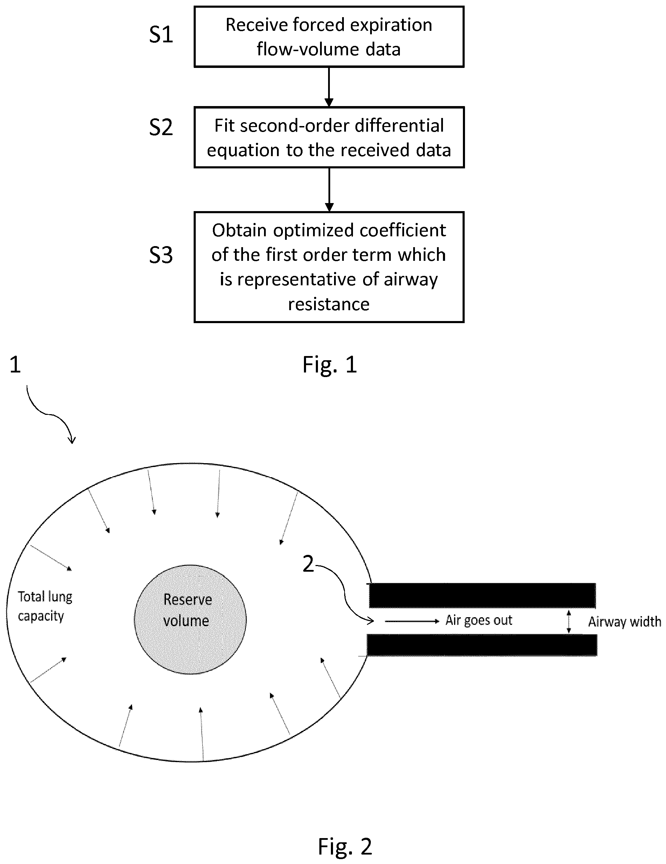

Referring to Fig. 1, a flow chart illustrating a method according to embodiments of the present invention is shown. Forced expiration flow-volume data collected by a spirometer is received (step S1). A second order differential equation in volume is fitted to the forced expiration flow-volume data (step S2). A parameter representative of airway resistance is determined in dependence upon a coefficient of a first order term in the differential equation (step S3).

*Collection of forced expiration flow-volume data*

In step S1, forced expiration flow-volume data collected by a spirometer is received. As will be described in more detail hereinafter, the data may be received, for example, by a processor or computer configured to communicate with the spirometer.

*Fitting the forced expiration flow-volume data*

In step S2, a second order differential equation is fitted to the received forced expiration flow-volume data.
During forced expiration, a test subject exhales forcefully from Total Lung Capacity (TLC) to Reserve Volume (RV) and the difference in volume is the Forced Vital Capacity (FVC).
Referring to Fig. 2, and without wishing to be bound by theory, it is thought that the process of forced expiration can be considered equivalent to a process of deflation of a balloon 1 through an outlet 2 from a first volume V1 to a second volume V2 which is less than the first volume V1. The first volume V1 can represent the Total Lung Capacity and the second volume V2 can represent the Reserve Volume. The air-flow during the deflation process may be opposed by the narrowing of the airways at an outlet 2 of the balloon 1.
This process of deflation may be modelled as a second order differential equation according to equation 1:

$$\ddot{x} + 2\zeta\omega_n\dot{x} + \omega_n^2 x = \delta(t) \qquad (1)$$

where $x$ is volume, $\dot{x}$ is the flow rate or flow, $\zeta$ (zeta) is a parameter representative of airway resistance, $\omega_n^2 x$ represents the elastic forces of the lungs and chest wall as well as expiratory muscles (i.e. the subjects own effort in executing the process of forced expiration), $t$ is time, and $\delta(t)$ is an impulsive force which occurs in time interval t resulting in Peak Expiratory Flow (PEF). The interval between t=0 and t (t having a specific value, e.g. t = t1) is very small (much less than one second). The value of x at $t$ = 0 is set as the FVC (the difference between total lung capacity or TLC and reserve volume or RV), the flow rate $\dot{x}$ at $t$ = 0 is set as 0, and the flow rate $\dot{x}$ at $t$ is set as the peak expiratory flow (PEF).
The impulsive force $\delta(t)$ can be modelled as a linear or non-linear function of time within the very short interval between 0 and t.
The action of the impulsive force can be represented by modeling $\dot{x}$ between time t=0 and t as a non-linear strictly increasing function of time that changes from 0 to PEF in the interval t=0 to t=t1 and may take the form of equation 2:

$$\dot{x}(t) = \frac{t}{\alpha\,(t_1 - \beta t)} \; where\; \beta = 1 + \frac{1}{\alpha\,PEF} \qquad (2)$$

The parameter $\alpha$ is the rate at which a subject generates a sudden pressure gradient to execute the forced expiratory manoeuvere.
The PEF is determined from the received flow-volume data as the maximum expiratory airflow and occurs at time t. The FVC is determined from the received data as the difference in volume at time t=0 and at the time t at which the forced expiration ends.

A cost function which characterises the closeness of the model solution and the measured flow and volume data is defined. The cost function preferably takes the form of equation 3:

$$J(\alpha, \zeta, \omega_n)$$

$$= \sum_{t=0}^{t_n} \left( V(t) - x(t) \right)^2 + (F(t) - \dot{x}(t))^2 \qquad (3)$$

where V(t) and F(t) are the received volume and flow values respectively. $x(t)$ and $\dot{x}(t)$ are calculated volume and flow values, respectively, at time t, as determined according to the second order differential equation, for example equation 1 with parameters $\alpha$, $\zeta$, $\omega_n$. The optimization or the fitting problem can then be formulated as follows:

$$\min_{\alpha, \zeta, \omega_n} J(\alpha, \zeta, \omega_n)$$

$$s.t \ \alpha, \omega_n > 0 \ and \ \zeta > 1$$

This optimization or fitting problem is then solved using any suitable method which aims to reduce or preferably to minimize the cost function, which may take the form of equation (3).

For example, in preferred embodiments, the optimization or fitting problem is solved using differential evolution to obtain an optimal value of $\zeta$. A tolerance parameter can be used as a criterion for convergence for the optimization procedure which can be configured to terminate under the condition of equation 5:

$$\frac{\bar{\mathcal{P}} \times \text{tol}}{\sigma(\mathcal{P})} > 1 \qquad (5)$$

The symbol $\mathcal{P}$ represents population, which refers to a group of candidate solutions of the cost function. In the examples described herein, tol=0.01, but the invention is not limited thereto and the skilled person will understand that the tolerance may be chosen as any suitable value which enables a solution to be found.

It will be also understood that any suitable fitting or optimization routine may be used, such as the Nelder-Mead method, genetic algorithms, a particle swarm method, a simulated annealing method.

*Determining a parameter representative of airway resistance*

In step S3, a parameter representative of airway resistance is determined in dependence upon a coefficient of a first order term in the differential equation.

Referring to equation 1, the parameter representative of airway resistance, $\zeta$, is determined by dividing the coefficient of the flow rate term $\dot{x}$ by $2\omega_n$, where the coefficient is provided as an outcome of the fitting process of step S2.

The parameter representative of airway resistance, $\zeta$, may also be referred to as an obstruction coefficient (OC) and it is a dimensionless parameter. This parameter is greater than 1, giving rise to an overdamped system, as the process of forced expiration does not involve oscillations of x, the volume, about a mean value. The second term $2\zeta\omega_n\dot{x}$ in equation 1 represents a resisting force to the deflation process and $\zeta$, or the obstruction coefficient (OC), dictates how quickly the deflation occurs. A low OC results in a faster deflation, whereas a high OC results in a slower deflation. An analogy can be drawn with forced expiration: a test subject having low airway resistance will take shorter time to complete the forced expiration manoeuvre than a test subject having high airway resistance.

The OC in equation 1 can thus be interpreted as representing the airway resistance. An abnormal airway resistance can indicate one or more of airway collapse, loss of elasticity of lung parenchyma and narrowing of airways due to smooth muscle contraction, mucosal thickening, fibrotic retraction and intraluminal secretions.

*Results*

The OC was determined based on pre-bronchodilator spirometry data in a population of 246 chronic obstructive pulmonary disease (COPD) subjects and 91 healthy subjects. The COPD cohort included subjects ranging from mild to severe airflow obstruction with 48 in GOLD I, 68 in GOLD II, 59 in GOLD III and 71 in GOLD IV stage (with reference to the Global Initiative for Obstructive Lung Disease classification for COPD stages).

Spirometry measurements were performed on each subject using a MasterScreen Pneumo spirometer (available from Vyaire Medical Inc., Illinois, USA). The measurements were performed according to guidelines as set out in Standardisation of spirometry, Miller et al., European Respiratory Journal 2005 26: 319-338. At least three acceptable manoeuvres were produced by each subject and the best manoeuvre was taken as the one with highest sum of FEV1 and FVC. On an average, it was observed that the subjects performed around 4-5 manoeuvres. These measurements were taken before bronchodilator was administered.

Following the spirometry measurements, further pulmonary function tests were performed including post-bronchodilator spirometry. Lung volumes and airway resistance were measured with a constant body plethysmograph, the MasterScreen Body (available from Vyaire Medical Inc., Illinois, USA), with a breathing frequency of 35 breaths per minute.

The flow-volume data were pre-processed by first converting the units to SI units, then extracting the data corresponding to the forced expiratory manoeuvre. For each subject the best manoeuvre was selected according to ATS/ERS guidelines and used in the subsequent analysis.

Equation 1 was fitted to the flow-volume data for each subject. Differential evolution was used to fit the model of equation 1 to the data and the output of the optimization process was the OC parameter.

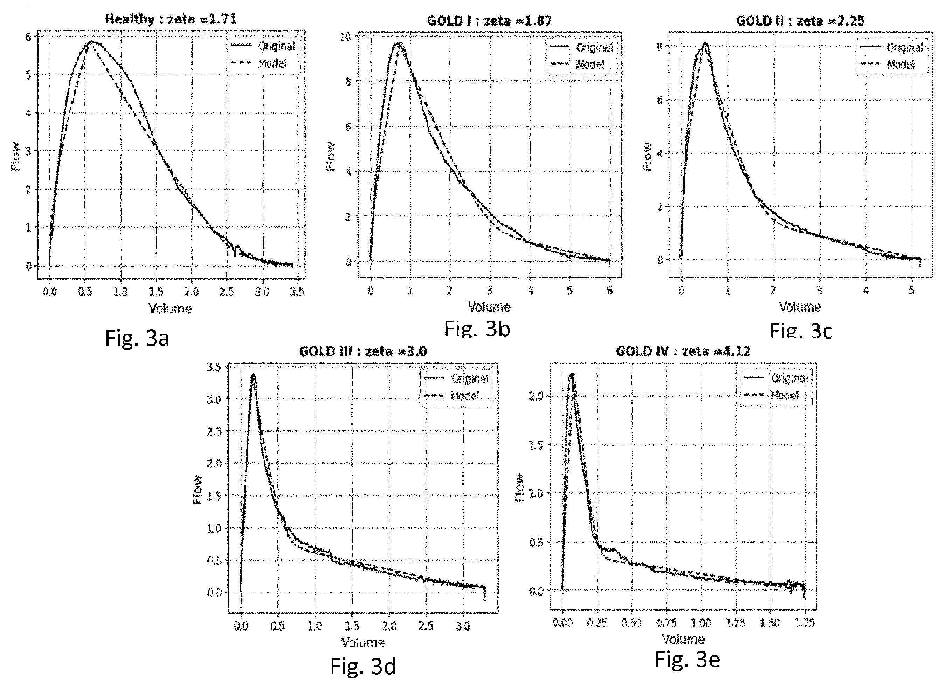

As can be seen from Fig. 3, the fitted second-order differential equations show a very good agreement with the flow-volume data obtained by the forced expiration test using the spirometer for healthy and COPD subjects. It can be seen that the model fits the data very well for both the period before the PEF (time to peak flow) and the period after the PEF (time from peak flow to end of expiration).

Table 1 shows the mean $\pm$ standard deviation of the dimensionless obstruction coefficient for the subject groups tested.

| Healthy | GOLD I | GOLD II | GOLD III | GOLD IV |
|---|---|---|---|---|
| 1.7±0.29 | 1.86 ± 0.3 | 2.15 ± 0.57 | 2.96 ± 0.85 | 3.85 ± 1.46 |

It is clear that the OC increases from Healthy and mild airflow obstruction to severe airflow obstruction (p<0.001).

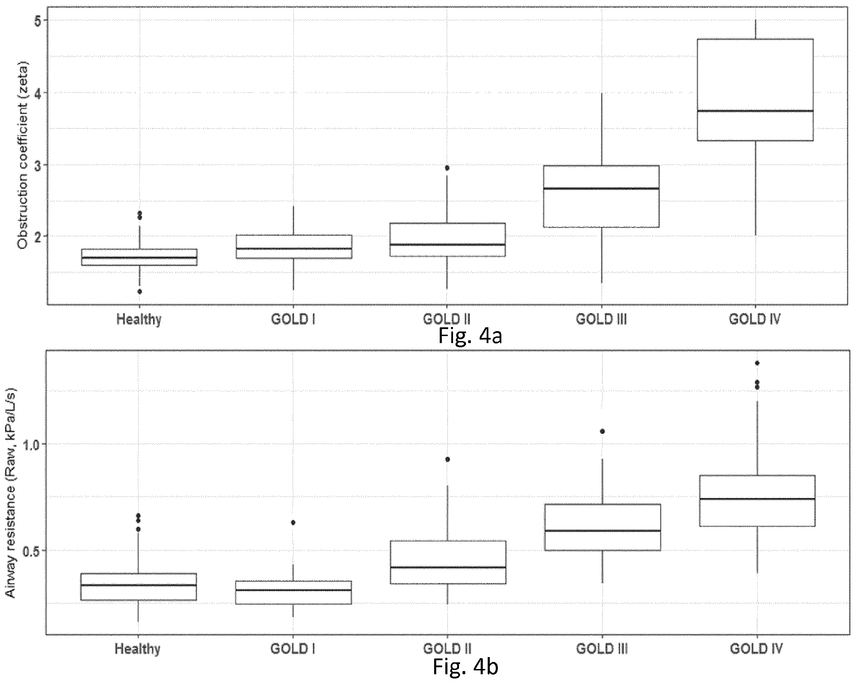

Referring to Fig. 4a, a box plot of the OC values obtained according to embodiments of the present invention is shown, and in Fig. 4b a box plot of airway resistance values (Raw) as measured in a body-plethysmography test is shown. It is clear that the OC values follow a very similar trend to that of the Raw values. Thus, the OC can be considered to be a parameter representative of the airway resistance, and this can be obtained using a simple, portable apparatus, the spirometer, which can be operated by a GP or nurse.

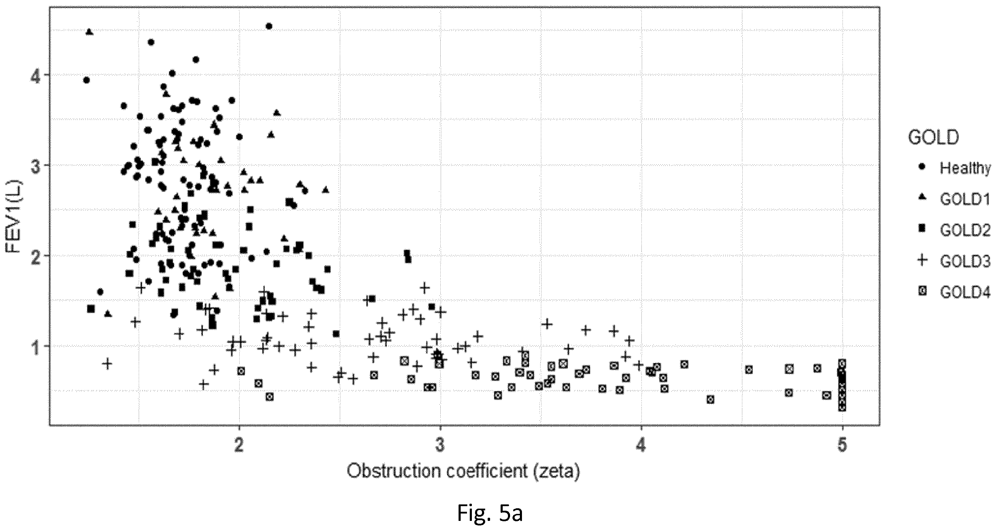

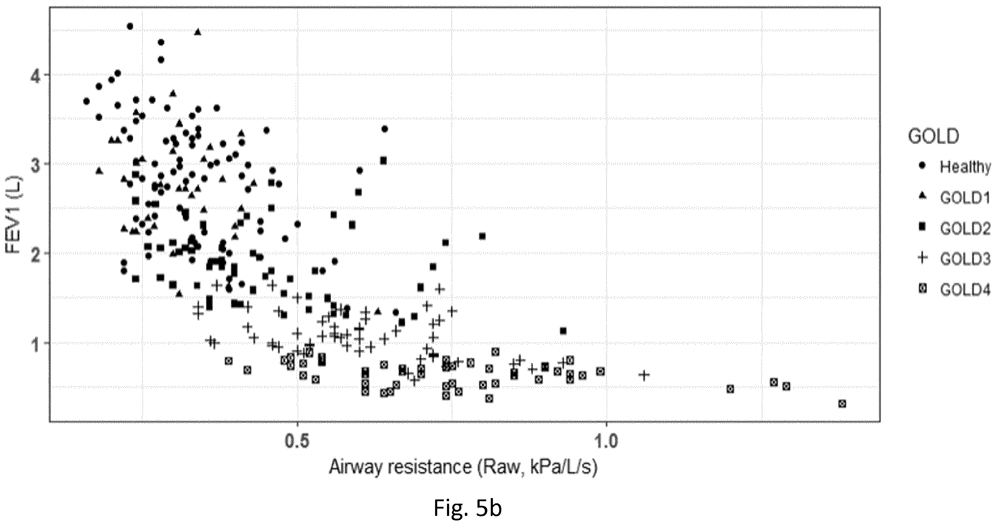

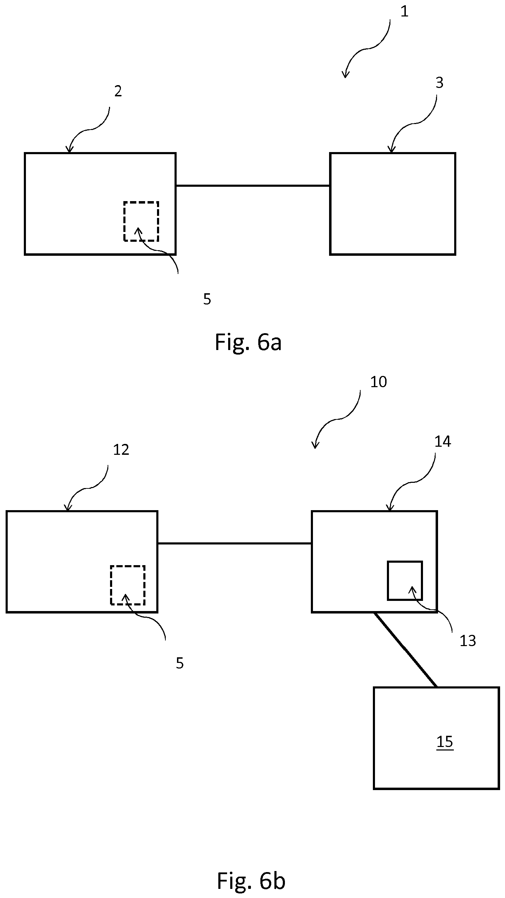

Referring to Fig. 5a, FEV1, the forced expiratory volume in 1 second, is plotted against OC as obtained according to embodiments of the present invention. Referring to Fig. 5b, FEV1 is plotted against Raw as measured in a body-plethysmography test. Both plots show again a very similar trend implying OC is representative of airway resistance. Referring to Fig. 6a, apparatus 1 is shown. Apparatus 1 comprises a spirometer 2 and a processor 3. The processor 3 is configured to receive forced expiration flow-volume data from the spirometer 2. The data may be received through transfer by a wired or wireless communication method, for example WiFi, Ethernet, Bluetooth, USB. In some embodiments, the processor 3 is comprised in the spirometer 2 and in this case the data is preferably received through a wired connection between the spirometer 2 and the processor 3.

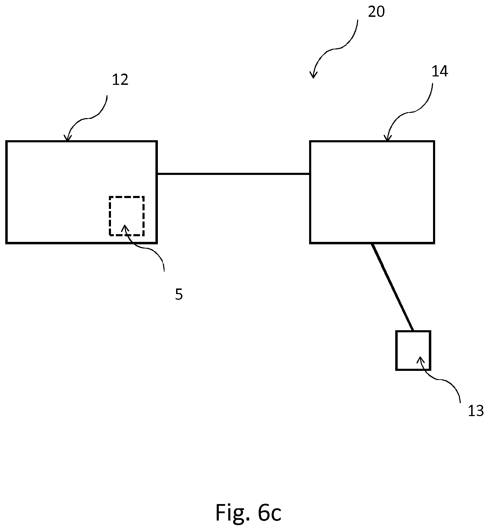

In some embodiments, the processor 3 is not comprised in the spirometer 2, and the data is received through a wired or wireless communication method. For example, referring to Fig. 6b, in apparatus 10, a processor 13 configured to receive forced expiration flow-volume data from a spirometer 12 may be comprised in a computer 14 and the spirometer 12 may be connected to the computer 14, for example using a USB cable (other wired or wireless connection methods are possible). The processor 13 may receive the data via an intermediate module running on the computer 14, which is configured to provide an interface with the spirometer 12. In preferred embodiments, the processor 13 takes the form of a software module or a calculation module configured to perform calculations locally or in the cloud. In some embodiments the processor 13 may be remote to the spirometer 12. For example, the processor 13 may be located at a server. The spirometer 12 may provide the data to the processor 13 directly through a wired or wireless connection, or indirectly. For example, referring to Fig. 6c, in apparatus 20, the spirometer 12 may be connected to the computer 14 and the computer 14 may provide the data to the processor 13 through a wired or wireless connection.

The processor 3 or 13 is configured to perform the steps of fitting a second order differential equation in flow to the data and determining a parameter representative of airway resistance in dependence upon a coefficient of a first order term in the differential equation. The parameter may be the obstruction coefficient as described hereinbefore.

The processor 3 or 13 is further configured to provide the parameter as output. For example, the processor 3 may provide the parameter to the spirometer 2 or 12 and the spirometer 2 or 12 may comprise a screen 5 which may display the parameter. The processor 13 may be comprised in a computer 14 (Fig. 6b) and the computer 14 may comprise or be connected to a display module 15. The display module 15 may display the parameter.

Alternatively to the examples provided, also other second order differential equations could be used, such as for example

$$\ddot{x} + 2\zeta\omega_n\dot{x} + \omega_n^2 x = 0$$

with

$$x(t1) = FVC - \Delta V$$

$$\dot{x}(t1) = PEF$$

where

$$\Delta V = \int_0^{t1} \dot{x}(t)$$

In the above wording, the effect of the impulsive force is neglected. The latter is effort dependent.

The invention is defined by the appended claims.

**Claims**

1. Apparatus (1,10,20) for determining a parameter representative of airway resistance, the apparatus comprising

   - a spirometer (2,12) for obtaining forced expiration flow-volume data,
   - a processor (3,13) configured to receive forced expiration flow-volume data from the spirometer, wherein the processor is configured to perform the steps of:

      (i) fitting a second order differential equation expressing the change of volume as function of time and the second differential of the volume as function of time to said flow-volume data and
      (ii) determining a parameter representative of airway resistance in dependence upon a coefficient of a first order term in the obtained differential equation, said determining the parameter being based only on the spirometer data,

   wherein the processor is further configured to output the parameter representative of the airway resistance.

2. Apparatus according to claim 1,

   wherein said fitting a second order differential equation in volume comprises fitting a second order differential equation having the form according to equation (1)

   $$\ddot{x} + 2\zeta\omega_n\dot{x} + \omega_n^2 x = \delta(t) \qquad\qquad (1)$$

   wherein, in equation (1), x is volume, $\dot{x}$ is the flow rate, $\zeta$ is a parameter representative of airway resistance, $\omega_n$ is a parameter representative of tissue elasticity and expiratory muscle force, $t$ is time, and $\delta(t)$ is an impulsive force which occurs in time t resulting in Peak Expiratory Flow (PEF) .

3. Apparatus according to any of claims 1 to 2, wherein said fitting comprises fitting the second order differential equation to at least one data point of a first set of flow-volume data corresponding to forced expiration before a point of peak expiratory flow.

4. Apparatus according to any of claims 1 to 3, wherein said fitting comprises fitting the second order differential equation to at least one data point of a second set of flow-volume data corresponding to forced expiration after a point of peak expiratory flow.

**5.** Apparatus according to any of claims 1 to 4, wherein the processor further is configured for determining a forced vital capacity based on the flow-volume data and using the forced vital capacity as a boundary condition of the second order differential equation and/or wherein the processor is configured for fitting the second order differential equation solely based on the flow-volume data.

**6.** Apparatus according to any of claims 1 to 5, wherein the processor further is configured for determining a peak expiratory flow value based on the flow-volume data and using the peak expiratory flow value as a boundary condition of the second order differential equation.

**7.** Apparatus according to any of claims 1 to 6, wherein the spirometer comprises the processor.

**8.** Apparatus according to any of claims 1 to 7, wherein the processor is a software module or wherein the processor is a cloud-based software module.

**9.** **A computer-implemented** method for analyzing forced expiration comprising

- receiving forced expiration flow-volume data collected by a spirometer;
- fitting a second order differential equation expressing the change of volume as function of time and the second differential of the volume as function of time to said flow-volume data; and
- determining a parameter representative of airway resistance in dependence upon a coefficient of a first order term in the differential equation, said determining the parameter being determining based only on the spirometer data, and
- outputting the parameter representative of the airway resistance.

**10.** A method according to claim 9, wherein the second order differential equation has the form according to equation (1) below:

$$\ddot{x} + 2\zeta\omega_n\dot{x} + \omega_n^2 x = \delta(t) \qquad\qquad (1)$$

wherein, in equation (1), x is volume, $\dot{x}$ is the flow rate, $\zeta$ is a parameter representative of airway resistance, $\omega_n$ is a parameter representative of tissue elasticity and expiratory muscle force.

**11.** A method according to claim 9 or 10, wherein the data comprises a first set of flow-volume data corresponding to forced expiration before a point of peak expiratory flow and fitting the second order differential equation to the data comprises fitting at least one data point in the first set and/or wherein the data comprises a second set of flow-volume data corresponding to forced expiration after a point of peak expiratory flow and fitting the second order differential equation to the data comprises fitting at least one data point in the second set.

**12.** A method according to any of claims 9 to 11, further comprising determining a forced vital capacity based on the received flow-volume data and using the forced vital capacity as a boundary condition of the second order differential equation and/or further comprising determining a peak expiratory flow value based on the received flow-volume data and using the peak expiratory flow value as a boundary condition of the second order differential equation.

**13.** A computer program product comprising instructions which, when executed by a computer, cause the computer to carry out the steps of a method according to any of claims 9 to 12.

**Patentansprüche**

**1.** Einrichtung (1,10,20) zum Bestimmen eines für einen Atemwegswiderstand repräsentativen Parameters, wobei die Einrichtung umfasst

- ein Spirometer (2,12) zum Erhalten von Flussvolumendaten einer forcierten Ausatmung,
- einen Prozessor (3,13), der konfiguriert ist, um Flussvolumendaten einer forcierten Ausatmung von dem Spirometer zu empfangen, wobei der Prozessor konfiguriert ist, um die Schritte durchzuführen zum:

(i) Anpassen einer Differentialgleichung zweiter Ordnung, welche die Volumenänderung in Abhängigkeit von Zeit, und das zweiten Differential des Volumens in Abhängigkeit von Zeit ausdrückt, an die Flussvolumendaten, und

(ii) Bestimmen eines für einen Atemwegswiderstand repräsentativen Parameters in Abhängigkeit von einem Koeffizienten eines Terms erster Ordnung in der erhaltenen Differentialgleichung, wobei das Bestimmen des Parameters nur auf den Spirometerdaten basiert, wobei der Prozessor weiter konfiguriert ist, um den für einen Atemwegswiderstand repräsentativen Parameter auszugeben.

2.  Einrichtung nach Anspruch 1, wobei das Anpassen einer Differentialgleichung zweiter Ordnung im Volumen Anpassen einer Differentialgleichung zweiter Ordnung umfasst, welche die Form gemäß Gleichung (1) aufweist

$$\ddot{x} + 2\zeta\omega_n\dot{x} + \omega_n^2 x = \delta(t) \qquad (1)$$

wobei in Gleichung (1) x Volumen ist, ẋ die Flussrate ist, $\zeta$ ein für einen Atemwegswiderstand repräsentativer Parameter ist, $\omega_n$ ein für Gewebeelastizität und Kraft der Muskulatur zur Ausatmung repräsentativer Parameter ist, t Zeit ist, und $\delta(t)$ eine Stoßkraft ist, die in der Zeit t auftritt, woraus sich die maximale Ausatmungsflussrate (PEF) ergibt.

3.  Einrichtung nach einem der Ansprüche 1 bis 2, wobei das Anpassen das Anpassen der Differentialgleichung zweiter Ordnung an mindestens einen Datenpunkt eines ersten Satzes von Flussvolumendaten umfasst, die forcierter Ausatmung vor einem Punkt des maximalen Ausatmungsflusses entsprechen.

4.  Einrichtung nach einem der Ansprüche 1 bis 3, wobei das Anpassen das Anpassen der Differentialgleichung zweiter Ordnung an mindestens einen Datenpunkt eines zweiten Satzes von Flussvolumendaten umfasst, die forcierter Ausatmung nach einem Punkt des maximalen Ausatmungsflusses entsprechen.

5.  Einrichtung nach einem der Ansprüche 1 bis 4, wobei der Prozessor weiter zum Bestimmen einer forcierten Vitalkapazität basierend auf den Flussvolumendaten und Verwenden der forcierten Vitalkapazität als Grenzbedingung der Differentialgleichung zweiter Ordnung konfiguriert ist und/oder wobei der Prozessor zum Anpassen der Differentialgleichung zweiter Ordnung ausschließlich basierend auf den Flussvolumendaten konfiguriert ist.

6.  Einrichtung nach einem der Ansprüche 1 bis 5, wobei der Prozessor weiter zum Bestimmen eines maximalen Ausatmungsflusswertes basierend auf den Flussvolumendaten und Verwenden des maximalen Ausatmungsflusswertes als Grenzbedingung der Differentialgleichung zweiter Ordnung konfiguriert ist.

7.  Einrichtung nach einem der Ansprüche 1 bis 6, wobei das Spirometer den Prozessor umfasst.

8.  Einrichtung nach einem der Ansprüche 1 bis 7, wobei der Prozessor ein Software-Modul ist, oder wobei der Prozessor ein Cloud-basiertes Software-Modul ist.

9.  Ein computerimplementiertes Verfahren zum Analysieren forcierter Ausatmung, umfassend

    - Empfangen von Flussvolumendaten forcierter Ausatmung, die durch ein Spirometer gesammelt werden;
    - Anpassen einer Differentialgleichung zweiter Ordnung, welche die Volumenänderung in Abhängigkeit von Zeit, und das zweiten Differential des Volumens in Abhängigkeit von Zeit ausdrückt, an die Flussvolumendaten; und
    - Bestimmen eines für einen Atemwegswiderstand repräsentativen Parameters in Abhängigkeit von einem Koeffizienten eines Terms erster Ordnung in der Differentialgleichung, wobei das Bestimmen des Parameters nur auf den Spirometerdaten bestimmt wird, und
    - Ausgeben des für einen Atemwegswiderstand repräsentativen Parameters.

10. Ein Verfahren nach Anspruch 9, wobei die Differentialgleichung zweiter Ordnung die Form gemäß der nachfolgenden Gleichung (1) aufweist:

$$\ddot{x} + 2\zeta\omega_n\dot{x} + \omega_n^2 x = \delta(t) \qquad (1)$$

wobei in Gleichung (1) x Volumen ist, ẋ die Flussrate ist, $\zeta$ ein für einen Atemwegswiderstand repräsentativer Parameter ist, $\omega_n$ ein für Gewebeelastizität und Kraft der Muskulatur zur Ausatmung repräsentativer Parameter ist.

**11.** Ein Verfahren nach Anspruch 9 oder 10, wobei die Daten einen ersten Satz von Flussvolumendaten umfassen, die forcierter Ausatmung vor einem Punkt eines maximalen Ausatmungsflusses entsprechen, und Anpassen der Differentialgleichung zweiter Ordnung an die Daten Anpassen mindestens eines Datenpunktes im ersten Satz umfasst und/oder wobei die Daten einen zweiten Satz von Flussvolumendaten umfassen, die forcierter Ausatmung nach einem Punkt eines maximalen Ausatmungsflusses entsprechen, und das Anpassen der Differentialgleichung zweiter Ordnung an die Daten Anpassen mindestens eines Datenpunkt im zweiten Satz umfasst.

**12.** Ein Verfahren nach einem der Ansprüche 9 bis 11, weiter umfassend das Bestimmen einer forcierten Vitalkapazität basierend auf den empfangenen Flussvolumendaten und Verwenden der forcierten Vitalkapazität als Grenzbedingung der Differentialgleichung zweiter Ordnung und/oder weiter umfassend das Bestimmen eines maximalen Ausatmungsflusswertes basierend auf den empfangenen Flussvolumendaten und Verwenden des maximalen Ausatmungsflusswertes als Grenzbedingung der Differentialgleichung zweiter Ordnung.

**13.** Ein Computerprogrammprodukt, das Anweisungen umfasst, die, wenn sie von einem Computer ausgeführt werden, den Computer dazu veranlassen, die Schritte eines Verfahrens nach einem der Ansprüche 9 bis 12 durchzuführen.

**Revendications**

**1.** Appareil (1, 10, 20) pour déterminer un paramètre représentatif de la résistance des voies respiratoires, ledit appareil comprenant :

- un spiromètre(2, 12) pour obtenir des données de débit-volume d'expiration forcée,
- un processeur (3, 13) configuré pour recevoir des données de débit-volume d'expiration forcée du spiromètre, dans lequel ledit processeur est configuré pour effectuer les étapes suivantes :

(i) ajuster une équation différentielle du second ordre exprimant le changement de volume en fonction du temps et la seconde dérivée du volume en fonction du temps auxdites données de débit-volume et
(ii) déterminer un paramètre représentatif de la résistance des voies respiratoires en fonction d'un coefficient d'un terme du premier ordre dans l'équation différentielle obtenue, ladite détermination du paramètre étant basée uniquement sur les données du spiromètre, dans lequel ledit processeur est en outre configuré pour sortir le paramètre représentatif de la résistance des voies respiratoires.

**2.** Appareil selon la revendication 1, dans lequel ledit ajustement d'une équation différentielle du second ordre en volume comprend l'ajustement d'une équation différentielle du second ordre ayant la forme selon l'équation (1)

$$\ddot{x} + 2\zeta\omega_n\dot{x} + \omega_n^2 x = \delta(t) \qquad (1)$$

dans lequel, dans l'équation (1), $x$ est le volume, $\dot{x}$ est le débit, $\zeta$ est un paramètre représentatif de la résistance des voies respiratoires, $\omega_n$ est un paramètre représentatif de l'élasticité des tissus et de la force musculaire expiratoire, $t$ est le temps, et $\delta(t)$ est une force impulsive qui se produit dans le temps t résultant en un débit expiratoire de pointe (PEF).

**3.** Appareil selon l'une quelconque des revendications 1 à 2, dans lequel ledit ajustement comprend l'ajustement de l'équation différentielle du second ordre à au moins un point de données d'un premier ensemble de données de débit-volume correspondant à une expiration forcée avant un point de débit expiratoire de pointe.

**4.** Appareil selon l'une quelconque des revendications 1 à 3, dans lequel ledit ajustement comprend l'ajustement de l'équation différentielle du second ordre à au moins un point de données d'un second ensemble de données de débit-volume correspondant à une expiration forcée après un point de débit expiratoire de pointe.

**5.** Appareil selon l'une quelconque des revendications 1 à 4, dans lequel le processeur est en outre configuré pour déterminer une capacité vitale forcée basée sur les données de débit-volume et utilisant la capacité vitale forcée comme condition limite de l'équation différentielle du second ordre et/ou dans lequel le processeur est configuré pour ajuster l'équation différentielle du second ordre uniquement basée sur les données de débit-volume.

**6.** Appareil selon l'une quelconque des revendications 1 à 5, dans lequel le processeur est en outre configuré pour déterminer une valeur de débit expiratoire de pointe basée sur les données de débit-volume et utilisant la valeur de débit expiratoire de pointe comme condition limite de l'équation différentielle du second ordre.

7. Appareil selon l'une quelconque des revendications 1 à 6, dans lequel le spiromètre comprend le processeur.

8. Appareil selon l'une quelconque des revendications 1 à 7, dans lequel le processeur est un module logiciel ou dans lequel le processeur est un module logiciel basé sur le cloud.

9. Méthode mise en œuvre par ordinateur pour analyser l'expiration forcée comprenant :

   - la réception de données de débit-volume d'expiration forcée collectées par un spiromètre ;
   - l'ajustement d'une équation différentielle du second ordre exprimant le changement de volume en fonction du temps et la seconde dérivée du volume en fonction du temps à lesdites données de débit-volume ; et
   - la détermination d'un paramètre représentatif de la résistance des voies respiratoires en fonction d'un coefficient d'un terme du premier ordre dans l'équation différentielle, ladite détermination du paramètre étant déterminée uniquement sur la base des données du spiromètre, et
   - la sortie du paramètre représentatif de la résistance des voies respiratoires.

10. Méthode selon la revendication 9, dans laquelle l'équation différentielle du second ordre a la forme selon l'équation (1) ci-dessous :

$$\ddot{x} + 2\zeta\omega_n\dot{x} + \omega_n^2 x = \delta(t) \qquad\qquad (1)$$

dans laquelle, dans l'équation (1), $x$ est le volume, $\dot{x}$ est le débit, $\zeta$ est un paramètre représentatif de la résistance des voies respiratoires, $\omega_n$ est un paramètre représentatif de l'élasticité des tissus et de la force musculaire expiratoire.

11. Méthode selon la revendication 9 ou 10, dans laquelle les données comprennent un premier ensemble de données de débit-volume correspondant à une expiration forcée avant un point de débit expiratoire de pointe et l'ajustement de l'équation différentielle du second ordre aux données comprend l'ajustement d'au moins un point de données dans le premier ensemble et/ou dans lequel les données comprennent un second ensemble de données de débit-volume correspondant à une expiration forcée après un point de débit expiratoire de pointe et l'ajustement de l'équation différentielle du second ordre aux données comprend l'ajustement d'au moins un point de données dans le second ensemble.

12. Méthode selon l'une quelconque des revendications 9 à 11, comprenant en outre la détermination d'une capacité vitale forcée basée sur les données de débit-volume reçues et utilisant la capacité vitale forcée comme condition limite de l'équation différentielle du second ordre et/ou comprenant en outre la détermination d'une valeur de débit expiratoire de pointe basée sur les données de débit-volume reçues et utilisant la valeur de débit expiratoire de pointe comme condition limite de l'équation différentielle du second ordre.

13. Produit de programme informatique comprenant des instructions qui, lorsqu'elles sont exécutées par un ordinateur, amènent l'ordinateur à effectuer les étapes d'une méthode selon l'une quelconque des revendications 9 à 12.

S1 | Receive forced expiration flow-volume data

S2 | Fit second-order differential equation to the received data

S3 | Obtain optimized coefficient of the first order term which is representative of airway resistance

Fig. 1

1

2

Total lung capacity

Reserve volume

Air goes out

Airway width

Fig. 2

Fig. 3a

Fig. 3b

Fig. 3c

Fig. 3d

Fig. 3e

Fig. 4a

Fig. 4b

Fig. 5a

Fig. 5b

EP 3 764 898 B1

Fig. 6a

Fig. 6b

Fig. 6c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010147305 A1

**Non-patent literature cited in the description**

- **OH et al.** Flow Decay: A Novel Spirometric Index to Quantify Dynamic Airway Resistance. *Respir. Care*, vol. 62 (7), 928-935

- **MILLER et al.** Standardisation of spirometry. *European Respiratory Journal*, 2005, vol. 26, 319-338